# EUROPÄISCHE PATENTSCHRIFT

(11) **EP 1 970 842 B1**
(45) Veröffentlichungstag und Bekanntmachung des Hinweises auf die Patenterteilung: **13.11.2019**
(21) Anmeldenummer: 08159421.0
(22) Anmeldetag: 23.08.2005
(51) Int. Cl.: A61M 15/00, G06M 1/08, B65D 83/14, G06M 1/04, G06M 1/24

(54) **Inhalier-Gerät**
Inhaler
Inhalateur

(30) Priorität: 10.11.2004 DE 102004054179; 18.07.2005 DE 102005033398
(43) Veröffentlichungstag der Anmeldung: 17.09.2008
(62) Teilanmeldung aus: 05792078.7
(73) Patentinhaber: Von Schuckmann, Alfred, 47627 Kevelaer (DE)
(72) Erfinder: Von Schuckmann, Alfred, 47627 Kevelaer (DE)
(74) Vertreter: Müller, Enno

(56) Entgegenhaltungen:
- EP-A- 0 480 488
- GB-A- 1 317 315
- US-A1- 2004 149 772
- US-A1- 2004 211 420
- US-B1- 6 752 153

## Beschreibung

Die Erfindung betrifft ein Handgerät zur portionierten Ausgabe sprühfähiger Substanzen nach den Merkmalen des Oberbegriffes des Anspruches 1.

Handgeräte der in Rede stehenden Art finden insbesondere Anwendung in der medizinischen Aerosol-Therapie zur Behandlung von Erkrankungen der Atemwege. Die in dem Handgerät-Gehäuse gehalterte, unter Druck stehende Kartusche beinhaltet das zu inhalierende Medikament, wozu zur Freigabe bzw. zum Ausstoß desselben eine axiale Verschiebung der Kartusche in dem Handgerät-Gehäuse erforderlich ist. Hierzu ist die Kartusche mit einem Ventil ausgestattet zur Abgabe einer vorbestimmten Medikamentenmenge. Das in der Regel die Kartusche nahezu vollständig umfassende Handgerät-Gehäuse besitzt weiter in üblicher Weise ein Mundstück und/oder einen Adapter zur Inhalation durch die Nase. Es besteht das Bedürfnis, dem Benutzer eine Zählvorrichtung anzubieten, welche die verbrauchte oder die in der Kartusche noch vorhandene Medikamentenmenge anzeigt. Da bei jeder Kartuschenbetätigung eine definierte Medikamentenmenge abgegeben wird, ist es bekannt, die Zählvorrichtung an die Axialverlagerung der Kartusche in dem Inhaler-Gehäuse zur Medikamentenabgabe zu koppeln. Mit jedem Öffnungshub der Kartusche wird eine ringförmige Skala gedreht. Läuft die Skalenscheibe um eine Achse um, die senkrecht zum Kartuschen-Hub steht (US 6,431,168), kann die dem Sichtfenster zugekehrte Mantelfläche, welche die Zahlenwerte trägt, nur sehr klein sein, wegen des nur kleinen Raumes unterhalb der Kartusche. Günstiger ist deshalb die ebenfalls vorbekannte Lösung, einen Skalenring vorzusehen, der um eine Achse umläuft, die mit der Kartuschenachse zusammenfällt (US 2004/0144798). Dort liegt die Drehebene des Skalenringes fest und vor einem Sichtfenster des Handgerät-Gehäuses.

Aus der US 2004/0149772 A1 ist ein Handgerät zur portionierten Ausgabe sprühfähiger Substanzen bekannt, bei welchem bei Bewegung der Kartusche ein Zählwerk betätigt wird. Die hierbei vorgesehenen zwei Zählringe bewegen sich bei einer Betätigung lediglich drehend relativ zu dem Gehäuse.

Ausgehend von dem dargelegten Stand der Technik beschäftigt sich die Erfindung mit der Aufgabenstellung, ein Handgerät zur portionierten Ausgabe sprühfähiger Substanzen anzugeben, das hinsichtlich der Erkennbarkeit einer Zählung günstig ausgebildet ist.

Diese Aufgabe ist beim Gegenstand des Anspruches 1 gelöst, wobei darauf abgestellt ist, dass ein in einem tellerförmigen Gehäuse (34) angeordnetes Schrittschaltwerk (11) mit Schaltgliedern (S, 28, 29, 16, 17, 20, 15, 21, 22, 13, 26), die um in Längsrichtung der Kartusche (3) liegende Achsen umlaufen, wobei eine Skala des Schrittschaltwerkes (11) als konzentrisch zu dem Ventilrohr (5) umlaufender Skalenring (26) gestaltet ist, der über ein Planetenrad-Getriebe (12) von einem ebenfalls konzentrisch zu dem Ventilrohr (5) umlaufenden, von einem Öffnungshub der Kartusche (3) angetriebenen Zahnkranz (16) schrittweise gedreht wird, wobei ein Schrittschaltfinger-Stern (S) vorgesehen ist, eine Nabe (29) des Schrittschaltfinger-Sterns (S) auf einer Stirnfläche eines handgerätgehäuseseitigen Stützabschnitts (9) für das Ventilrohr (5) sitzt, und bei Durchführung eines Betätigungshubs der Kartusche und damit einhergehender Vertikalverlagerung der Kartusche (3) in Richtung auf den Stützabschnitt (9) über einen Kartuschenkopf (4) mitgeschleppt wird, und ein zentrales Loch (38) zum Durchtritt des Ventilrohrs (5) besitzt, welches Loch (38) sich am kartuschenseitigen Ende zum Eintritt einer an der Kartuschen-Öffnungsseite vorstehenden Kragens (41) verbreitert, hinter dessen Bereich eine federnde Verrastung zwischen Schrittschaltwerkgehäuse (34) und Kartusche (3) erfolgt, das Schrittschaltwerkgehäuse (34) im Handgerätgehäuse (2) verrastet ist, derart, dass es noch um den Öffnungshub einwärts verlagerbar ist und bei Durchführung eines Betätigungshubs der Kartusche (3) und damit einhergehender Vertikalverlagerung der Kartusche (3) in Richtung auf den Stützabschnitt (9) des Schaltwerkgehäuses (34) über einen Kartuschenkopf (4) mitgeschleppt wird, dies unter Relativverlagerung des Schrittschaltwerkgehäuses (34), des Planetenrad-Getriebes (12) und des Skalenringes (26) zu dem Schrittschaltfinger-Stern (S), welcher eine Abstützung auf dem Stützabschnitt (9) erfährt.

Es ist ein mechanisch arbeitendes Schrittschaltwerk vorgesehen, welches aufgrund der gewählten Anordnung verschiedener Schaltglieder eine günstige, raumsparende Bauform aufweisen kann. Es ist weiter eine weitere Bauraumoptimierung erreicht, da der ohnehin unterhalb der Kartusche in dem Handgerät-Gehäuse vorhandene Freiraum funktionell mit einbezogen ist in die Bedienungskontrolle.

Das Schrittschaltwerk weist einen konzentrisch zum Ventilrohr umlaufenden Ring mit außenseitiger Skala auf, der über ein Planetenrad-Getriebe von einem ebenfalls konzentrisch zum Ventilrohr umlaufenden, vom Öffnungshub der Kartusche angetriebenen Zahnkranz schrittweise gedreht wird. Der Skalenring ist unterhalb der öffnungsseitigen Stirnwand der Kartusche in Überlappung zum Kartuschen-Ventilrohr angeordnet und weist insofern bevorzugt einen an den Kartuschendurchmesser angepassten Durchmesser auf. Dies bietet eine große Skalenlänge, nämlich in etwa entsprechend dem Kartuschenmantelumfang. Die Drehbewegung des Skalenrings ist abgezweigt aus der Relativverlagerung der Kartusche durch das Schrittschaltwerk, dessen Schaltfinger auf einer Bahn rund um das Kartuschen-Ventilrohr arbeitet. Der Schaltfinger ist hierbei bevorzugt Teil des integral aufgebauten Schrittschaltwerkes und findet Abstützung in der Betriebsstellung des Handgerätes im Bereich des gehäuseseitigen Ventilrohr-Aufnahmeabschnittes, welcher zugleich das Gegenlager für das Ventilrohr zur Ventilöffnung ausformt. Es ist ein Stützabschnitt für den Schaltfinger im Gehäuse gebildet. Bei einer möglichen Entnahme des Schrittschaltwerkes aus dem Gehäuse ist wegen des dann fehlenden Stützabschnittes für den Schaltfinger eine versehentliche kartuschenunabhängige Betätigung des Schrittschaltwerkes nicht möglich. Das Planetenrad-Getriebe gibt bevorzugt den Drehwinkel untersetzt an den Skalenring weiter. Entsprechend sind auf dem Skalenring angepasst an das Kartuschenvolumen bspw. 200 oder 300 Hubstöße anzeigbar.Weiter ist bevorzugt, dass die auf der äußeren Mantelfläche des Skalenrings angeordnete und vor den Sichtfenstern des Gehäuses laufende Skaleneinteilung des Skalenrings jeweils mehreren Einzel-Drehschritten des Planetenrades entspricht. Diesbezüglich erweist es sich weiterhin als vorteilhaft, dass hinsichtlich der Untersetzung ein Einzel-Drehschritt des Planetenrades nach Durchlauf mehrerer Einzel-Drehschritte des Sonnenrades erfolgt. Das Planetenrad steht radial weiter außen mit einem Zahnkranz in Eingriff. Letzterer weist mindestens einen von der unteren Randkante schräg aufwärts gerichtet ausgehenden Schlitz zum Eintritt des Schrittschaltfingers auf, um hierüber die Schaltrichtung des Fingers zum schrittweisen Weiterdrehen der sonnenradseitigen Scheibe vorzugeben.Das Schrittschaltwerkgehäuse mit den weiteren Schaltgliedern wird relativ zu dem Schrittschaltfinger-Stern verlagert, dies unter Bewegung der Schrittschaltfinger in die senkrecht zur Längsachse ausgerichteten Ebene, was das Weiterschalten des Zählwerkes bewirkt. Die abgetauchte Skala ist eine Art Anzeigeelement für den richtigen Funktionsablauf. Nach einem Loslassen der Kartusche eilt diese aufgrund der integrierten Ventilfeder zurück in ihre Grundstellung, was durch die nunmehr fehlende Beaufschlagung des Schrittschaltwerkgehäuses ein Nacheilen desselben bewirkt. Dies ist durch die vorgespannten Schrittschaltfinger erreicht, die ihre ursprüngliche, schräg aufwärts gerichtete Position selbsttätig wieder einnehmen und hierbei die Schaltglieder sowie das Schrittschaltwerkgehäuse in ihre Ursprungslage drücken. Entsprechend ist das Schrittschaltwerk hinsichtlich der Rückverlagerung in eine Grundstellung entkoppelt von der Kartusche, aber kontrollierbar durch die wiederkommende Skala. Es verfügt über eine durch die Schrittschaltfinger gebildete Rückstellfeder. Die axiale Länge des gesamten Schrittschaltwerkgehäuses ist weiter bevorzugt angepasst an die Erstreckungslänge des Ventilrohres, demzufolge letzteres in der entnommenen Stellung aus dem Handgerätgehäuse von dem Schrittschaltwerkgehäuse weiter umfasst in einer geschützten Stellung verbleibt. Alternativ kann das als Flachteller gestaltete Schrittschaltwerkgehäuse im Handgerätgehäuse verrastet sein derart, dass es noch um den Öffnungshub einwärts verlagerbar ist. So kann das Schrittschaltwerkgehäuse bspw. bei einer Erstbestückung des Handgerätes zusammen mit der Kartusche in das Handgerätgehäuse eingeschoben werden, woraufhin das Schrittschaltwerkgehäuse abschließend eine in dem Gehäuse vorgesehene Rast hintergreift. Aus dieser Grundstellung heraus ist das Schrittschaltwerk über die Kartuschenhubbewegung weiterhin einwärts verlagerbar. In einer Weiterbildung des Erfindungsgegenstandes ist eine doppelte Verrastung des als Flachteller gestalteten Schrittschaltwerkgehäuses vorgesehen derart, dass neben der Verrastung am Kragen der Kartusche eine zweite stärkere Verrastung im Handgerätgehäuse erfolgt. Vor einer Erstbenutzung ist es notwendig, das Handgerätgehäuse mit der Kartusche zu bestücken. Nachstehend ist die Erfindung anhand der beigefügten Zeichnung, welche lediglich verschiedene Ausführungsbeispiele darstellt, näher erläutert. Es zeigt:
- Fig. 1: in perspektivischer Explosionsdarstellung das Schrittschaltwerk für das erfindungsgemäße Handgerät;
- Fig. 2: einen Querschnitt durch das Schrittschaltwerk;
- Fig. 3: in einer Längsschnittdarstellung das Handgerät in einer ersten Ausführungsform, mit einem an einer Kartusche verrasteten, schematisch dargestellten Schrittschaltwerk;
- Fig. 4: eine der Fig. 3 entsprechende Schnittdarstellung, jedoch eine zweite Ausführungsform betreffend, bei welcher das schematisch dargestellte Schrittschaltwerk am Gehäuse verrastet ist;
- Fig. 4a: eine dritte Ausführungsform in einer Schnittdarstellung gemäß Fig. 4, bei welcher die Kartusche in dem Handgerätgehäuse gegen Abziehen blockiert ist;
- Fig. 5: in Längsschnittdarstellung das Handgerät in einer weiteren Ausführungsform, nach Trennen eines oberen Gehäuseteils von einem Mundstück.

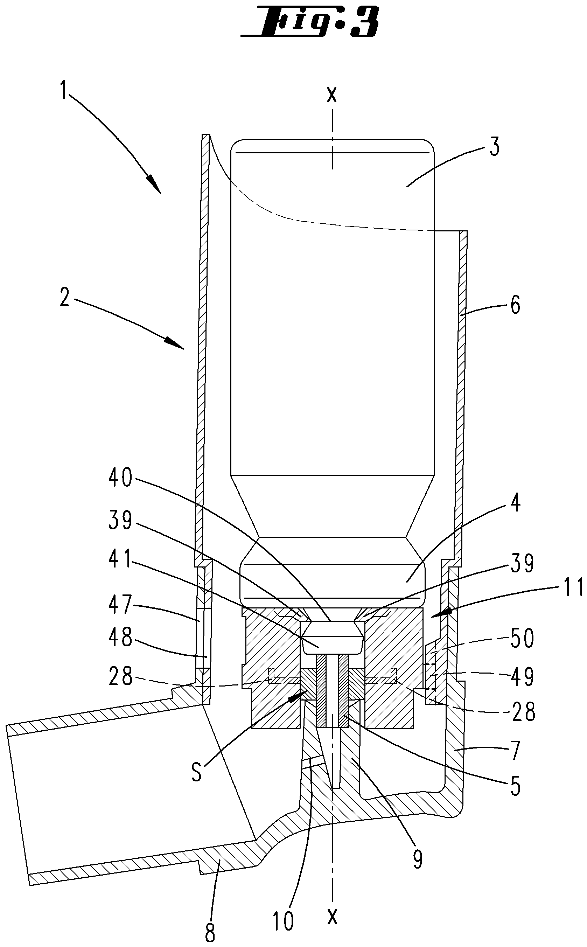

Das in Fig. 3 in einer schematischen Schnittdarstellung gezeigte Handgerät 1 dient zur portionierten Ausgabe sprühfähiger Substanzen, insbesondere von Inhaliermedikamenten.

Hierzu weist das Handgerät 1 zunächst ein Handgerätgehäuse 2 auf, in welches eine die sprühfähige Substanz beinhaltende Kartusche 3 einsetzbar ist. Diese Kartusche 3 ist in dem Gehäuse 2 axial verschiebbar.

In üblicher Weise weist der Kartuschenkopf 4 ein zentrales, sich koaxial zur Kartusche 3 erstreckendes Ventilrohr 5 auf. Über letzteres wird eine Medikamentenausgabe durch eine axiale Relativbewegung zwischen Kartusche 3 und Handgerät-Gehäuse 2 erreicht.

Das Handgerät-Gehäuse 2 ist zweigeteilt und besteht im Wesentlichen aus zwei übereinander angeordneten Ringteilen 6 und 7, von welchen das obere Ringteil 6 schaftartig ausgeformt ist und das untere Ringteil 7 ein etwa quer zur Schafterstreckung ausgerichtetes Mundstück 8 aufweist. Letzteres ist durch eine nicht dargestellte Abdeckkappe verschließbar.

Das Ventilrohr 5 der Kartusche 3 stützt sich in einem zugeordneten rohrförmigen Stützabschnitt 9 innerhalb des unteren Ringteiles 7 ab, dies bei axialer Beweglichkeit der Kartusche 3 innerhalb des die Kartusche 3 umgebenden, schaftartigen Ringteils 6.

Der das Ventilrohr 5 der Kartusche 3 klemmend aufnehmende, innerhalb des unteren Gehäuse-Ringteils 7 ausgeformte Stützabschnitt 9 ist mit einem gegenüber einen das Ventilrohrende aufnehmenden Abschnitt durchmesserverringerten Strömungskanal 10 versehen, welcher strömungstechnisch in Verbindung steht mit dem Ventilrohr 5, wobei das dem Ventilrohr 5 abgewandte Ende des Strömungskanals 10 in Richtung auf das Mundstück 8 weist.

Die beiden Ringteile 6 und 7 sind in dem dargestellten Ausführungsbeispiel steckbar miteinander verbunden. Alternativ können die beiden Teile auch über ein Gewinde, bspw. über ein Grobgewinde mit hoher Steigung, miteinander verbunden sein.

Die Anordnung der Kartusche 3 in dem Handgeräte-Gehäuse 2 ist so gewählt, dass der Kartuschenkopf 4 in dem Gehäuse 2 etwa auf Höhe des Verbindungsbereiches zwischen Ringteil 6 und Ringteil 7 platziert ist.

Zentral unterhalb der öffnungsseitigen Stirnwand der Kartusche 3 ist in Überlappung zum Kartuschen-Ventilrohr 5 ein Schrittschaltwerk 11 angeordnet. Dieses dient zum Registrieren und Anzeigen der durchgeführten Ausgabebetätigungen, dies in Abhängigkeit von den durchgeführten Öffnungshüben der Kartusche 3.

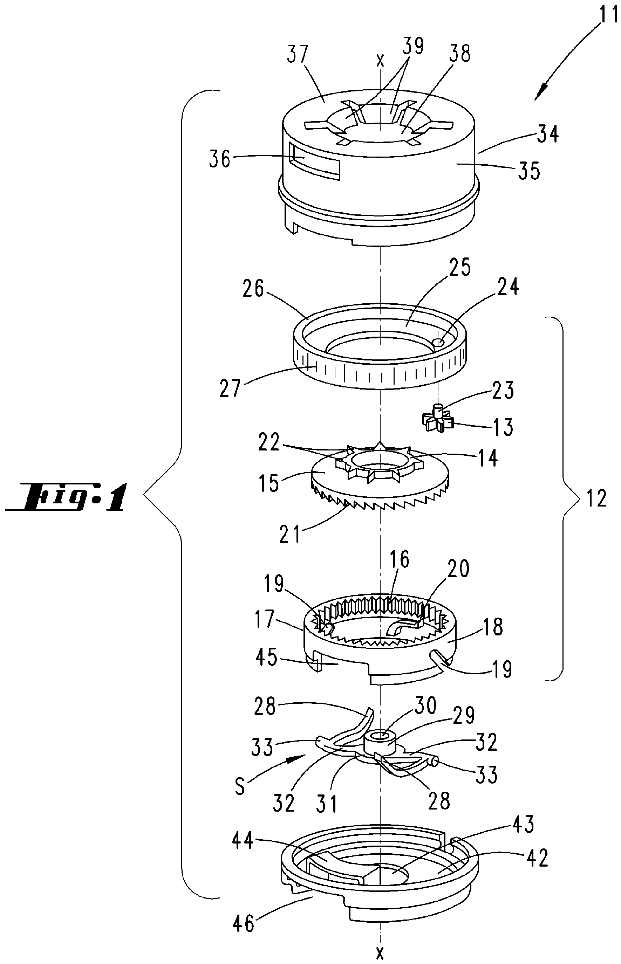

Das Schrittschaltwerk 11 ist in Fig. 1 in einer perspektivischen Explosionsdarstellung gezeigt. Zentraler Bestandteil des Schrittschaltwerks 11 ist ein Planetenrad-Getriebe 12, bestehend aus einem Planetenrad 13, einem Sonnenrad 14, welches auf einer unterseitig gezahnten Scheibe 15 sitzt und einem mit dem Planetenrad 13 zusammenwirkenden Zahnkranz 16. Letzterer ist wandungsinnenseitig eines nicht drehbar gehalterten, rohrabschnittförmigen Ringes 17 ausgeformt. Die Mantelwandung 18 des Ringes 17 ist in diametral gegenüberliegenden Bereichen durchsetzt von schräg aufwärts in Schaltrichtung gerichtet ausgehenden Schlitzen 19, die nach unten zur dem Zahnkranz 16 abgewandten Ringkante hin offen auslaufen.

Der Zahnkranz 16 erstreckt sich in axialer Richtung etwa über die halbe Höhe des Ringes 17, dessen Mantelwandung 18 zur dem Zahnkranz 16 abgewandten Ringstirnkante hin abgestuft, sich radial verjüngend ausgebildet ist.

Unterhalb des Zahnkranzes 16 ist innenseitig der Mantelwandung 18 des Ringes 17 ein Rastfinger 20 angeformt. Dieser ist mit Bezug auf einen Grundriss des Ringes gegenüber dem Zahnkranz 16 nach radial innen versetzt; greift entsprechend in einen zum Zahnkranz 16 nach radial innen eingezogenen Kreisraum. Weiter ist die Anordnung des in etwa in Vertikalrichtung elastisch ausgebildeten Rastfingers 20 so gewählt, dass dieser etwa in eine durch die unteren Randkanten des Zahnkranzes 16 aufgespannte Horizontalebene eingreift.

Der Durchmesser der das Sonnenrad 14 tragenden Scheibe 15 ist geringfügig kleiner gewählt, als der Innendurchmesser des Ringes 17 im Bereich des Zahnkranzes 16. Sonnenrad 14 und Scheibe 15 sind bevorzugt einstückig, materialeinheitlich ausgebildet.

Unterseitig der Scheibe 15 ist eine randbezogen umlaufende Sägezahnung 21 vorgesehen, in welche der Rastfinger 20 des Ringes 17 eingreift.

Das Sonnenrad 14 weist eine grobe Verzahnung auf. So sind in dem dargestellten Ausführungsbeispiel über den Umfang des Sonnenrades 14 acht Sonnenrad-Zähne 22 gleichmäßig verteilt ausgeformt. Diese Zähne 22 wirken im Zuge der Sonnenrad-Umdrehung mit dem in derselbe Ebene zwischen dem Sonnenrad 14 und dem Zahnkranz 16 des Ringes 17 angeordneten Planetenrad 13 zusammen.

Das Planetenrad 13 besitzt einen einseitig nach oben, d. h. von der Scheibe 15 des Sonnenrades 14 weg abragenden Achszapfen 23. Dieser ist in einer Bohrung 24 im Bereich eines scheibenartig nach radial innen weisenden Kragens 25 eines Skalenringes 26 drehbar gefasst. Der Skalenring 26 ist mantelaußenwandig mit einer umlaufenden Skaleneinteilung 27 versehen, wobei die Skaleneinteilung jeweils mehreren Einzel-Drehschritten des den Skalenring 26 weiterführenden Planetenrades 13 entspricht.

Die schrittweise Verlagerung des Sonnenrades 14 bzw. der einstückig hiermit ausgeformten Scheibe 15 erfolgt über etwa vertikal federnd ausweichbar ausgebildete Schrittschaltfinger 28. Diese greifen unterseitig in die Sägezahnung 21 der Scheibe 15.

Die Schrittschaltfinger 28 sind mit Bezug zu der Hauptachse x des gesamten Schrittschaltwerkes 11 diametral gegenüberliegend angeordnet. Hierzu ist zunächst ein zylinderförmiger Zentralkörper in Form einer Nabe 29 vorgesehen mit einer zentralen axialen Durchgangsbohrung 30. Deren Durchmesser ist geringfügig größer bemessen als der Außendurchmesser des diese Durchgangsbohrung 30 zu durchsetzenden Kartuschen-Ventilrohres 5.

Fußseitig geht die Nabe 29 über in einen radial erweiterten Kragen 31. An diesem sind diametral gegenüberliegend in Radialrichtung abragende Führungsabschnitte 32 angeformt, die jeweils im Bereich ihrer freien Enden einen in dem zugeordneten Schlitz 19 des Ringes 17 einliegenden Führungszapfen 33 ausformen.

Die Schrittschaltfinger 28 wurzeln jeweils mit einem Horizontalabschnitt an den Führungsabschnitten 32 unter Belassen der über den horizontalen Abschnitt radial außen abragenden Führungszapfen 33. Die von den Horizontalabschnitten abragenden Schrittschaltfinger 28 gehen schräg aufwärts gerichtet aus, etwa unter Einschließen eines Winkels von 45 Grad zur Horizontalen, angepasst an die Schrägung der Schlitze 19 im Ring 17. Der so gebildete Schrittschaltfinger-Stern trägt das Bezugszeichen S.

Der Schrittschaltfinger-Stern S, der den inneren Zahnkranz 16 aufweisende Ring 17, die einstückig mit dem Sonnenrad 14 ausgeformte Scheibe 15 sowie der Skalenring 26 sind konzentrisch zueinander auf der Achse x ausgerichtet, wobei die Höhe des Ringes 17 so gewählt ist, dass sowohl der Schrittschaltfinger-Stern S als auch das Sonnenrad 14 samt Scheibe 15 in diesem aufgenommen sind.

Das gesamte Planeten-Getriebe 12, sowie der Schrittschaltfinger-Stern S und der Skalenring 26 sind aufgenommen in einem topfartigen Schrittschaltwerkgehäuse 34 mit einem Außendurchmesser, welcher an den Außendurchmesser der Kartusche 3 angepasst ist.

Das Gehäuse 34 besitzt eine Mantelwandung 35. Diese weist ein Sichtfenster 36 auf, durch welches die Skaleneinteilung 27 des Skalenrings 26 erkennbar ist.

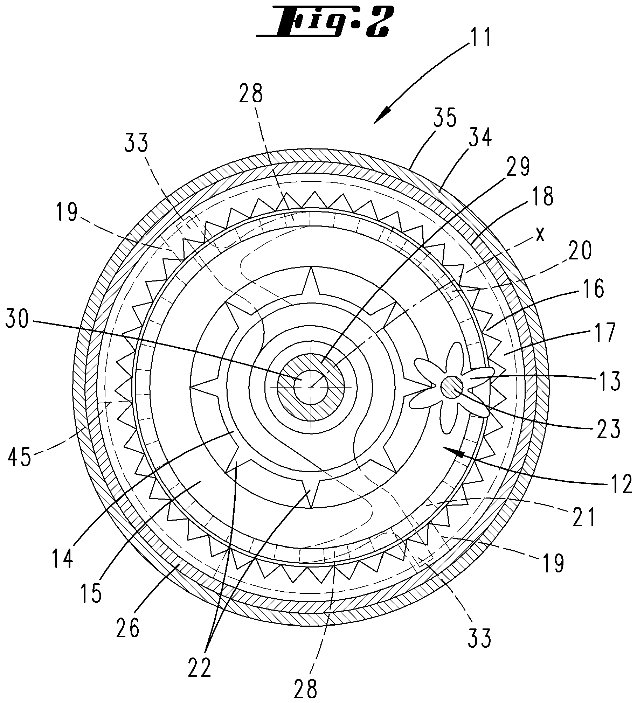

Die Gehäusedecke 37 besitzt eine zentrale Durchbrechung 38, welche in der in den Figuren 1 bis 3 gezeigten Ausführungsform umfasst ist von konisch zum Gehäuseinnern hin zulaufenden Rast-Federzungen in der in den Figuren 1 bis 3 gezeigten Ausführungsform 39. Der Durchbrechungs-Durchmesser ist angepasst an einen Durchmesser eines Taillenabschnittes 40 eines über die öffnungsseitige Stirnwand der Kartusche 3 zentral überragenden Kragens 41, aus welch letzterem das Ventilrohr 5 auswächst.

Der Gehäuseboden 42 ist gebildet durch ein gesondertes Teil. Dieses ist unter Aufnahme der vorbeschriebenen Schrittschaltwerk-Einzelteile mit dem Gehäuse 34 verbunden, so bspw. mit diesem verschweißt oder über eine Presspassung an diesem gehaltert.

Der tellerartige Gehäuseboden 42 besitzt eine zentrale Bohrung zum Durchtritt des Ventilrohres 5. Des Weiteren ist auf dem Gehäuseboden 42 ein Raststück 44 ausgeformt, welches zur lageorientierten Fesselung des Ringes 17 in eine entsprechend in dessen Mantelwandung 18 ausgeformte, fensterartige Ausnehmung 45 greift.

Im selben Winkelbereich, in welchem das Passstück 44 auf dem Boden angeordnet ist, weist die Mantelaußenwandung des Gehäusebodens 42 einen Freischnitt 46 auf. Diese ist im Einbauzustand dem Bereich des Ausgangsquerschnittes des Strömungskanals 10 im unteren Ringteilgehäuse 7 zugeordnet.

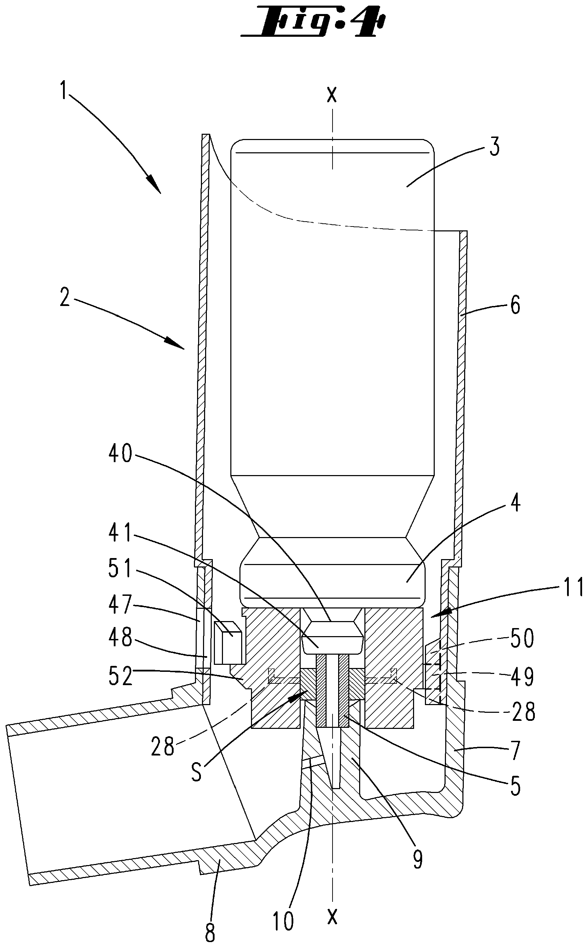

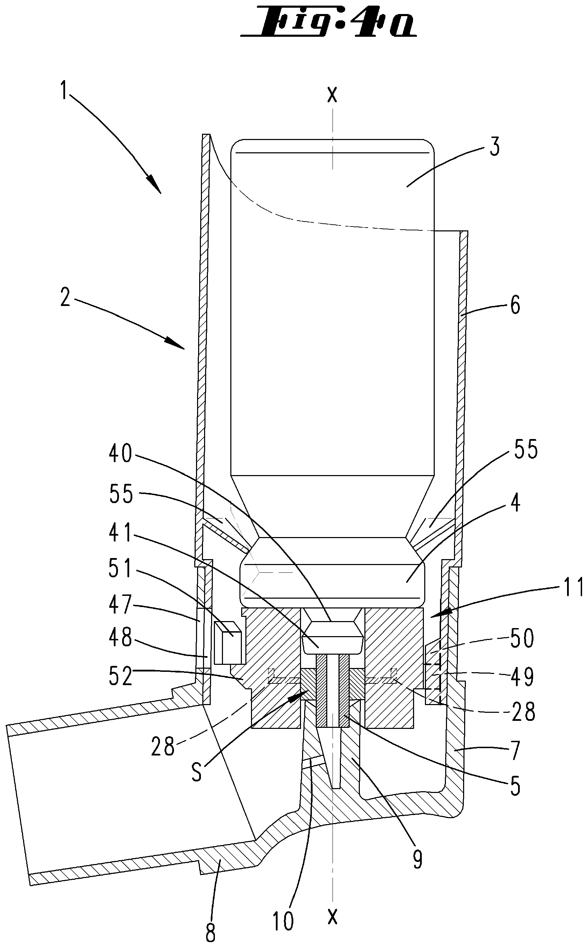

Die Funktionsweise des Schrittschaltwerkes 11 ist unabhängig von der anhand der Figuren 3 und 4 noch zu beschreibenden grundsätzlichen Anordnung wie folgt:
Die Schaltglieder (Schrittschaltfinger-Stern S, Ring 17, Scheibe 15, Planetenrad 13 und Skalenring 26) wie auch das Zählwerk-Gehäuse 34 mit dem Gehäuseboden 42 sind auf Achsen angeordnet, die sich in Längsrichtung der Kartusche 3 erstrecken. So sind mit Ausnahme des Planetenrades 13 alle weiteren Bauteile des Schrittschaltwerkes 11 auf der Kartuschenlängsachse x - x positioniert.

Das Schrittschaltwerk 11 ist entsprechend konzentrisch zum Ventilrohr 5 im Schatten der Kartusche 3 innerhalb des Gehäuses 2 angeordnet, dies konkret in dem zwischen Kartuschenkopf 4 und Stützabschnitt 9 des Handgerät-Gehäuses 2 belassenen Bauraum. Das Schrittschaltwerk 11 stützt sich mit der in dem Schaltwerkgehäuse 34 zentral gelagerten Nabe 29 des Schrittschaltfinger-Sterns S auf der Stirnfläche des Stützabschnitts 9 des Inhaliergehäuses 2 ab. Die zentrale Achse x des Schnittschaltwerks 11 wird von der Körperachse der Kartusche 3 übernommen. Das die Nabe 29 durchsetzende Ventilrohr 5 bietet eine zusätzliche Zentrierung der gesamten Schrittschaltwerk-Einheit.

Bei Durchführung eines Betätigungshubs der Kartusche 3 und damit einhergehender Vertikalverlagerung derselben in Richtung auf den Stützabschnitt 9 wird das Schaltwerkgehäuse 34 über den Kartuschenkopf 4 mitgeschleppt, dies unter Relativverlagerung des Zählwerk-Gehäuses 34, des Planetenrad-Getriebes 12 und des Skalenringes 26 zu dem Schrittschaltfinger-Stern S, welche eine Abstützung auf dem Stützabschnitt 9 erfährt. Infolgedessen bewirken die sich spannenden Schrittschaltfinger 28 weiter unterstützt durch Drehaufgleiten des Schrittschaltfinger-Sterns S in den mantelwandseitigen Schlitzen 19 des Ringes 17 einen schrittweisen Drehvorschub der sägeverzahnten Scheibe 15. Einhergehend damit dreht sich das Sonnenrad 14 um denselben Winkelbetrag. Die Schrittschaltfinger 28 bewegen sich hierbei aus der schrägen Ausrichtung in Richtung auf eine senkrecht zur Längsachse x - x ausgerichtete Ebene.

Dadurch bedingt, dass das Sonnenrad 14 lediglich acht gleichmäßig über den Umfang verteilt angeordnete Zähne aufweist, führt nicht jede Schritt-Drehbewegung des Sonnenrades 14 zwangsläufig zu einer Drehbewegung des Planetenrades 13. Vielmehr wird die Drehung des Planetenrades 13 um dessen Achse und eine die damit einhergehende Drehverlagerung des Skalenringes 26 erst nach mehreren Einzel-Drehschritten des Sonnenrades 14 durchgeführt.

Gemäß der Darstellung in Fig. 3 ist das gesamte Schrittschaltwerk 11 mittels des Zählwerk-Gehäuses 34 an dem kartuschenkopfseitig vorragenden Kragen 41 rastend festlegbar. Zugeordnet zu dem gehäuseseitigen Sichtfenster 36 weisen die zugeordneten Abschnitte der Gehäuseringteile 6 und 7 gleichfalls Sichtfenster 47, 48 auf, welche durch die gewählte Position, zugewandt dem Mundstück 8 des Gehäuses 2, im Blickfeld des das Handgerät 1 bedienenden Benutzers liegen. Zur lageorientierten Einführung des Schrittschaltwerkes 11 ist dieses mit einem radial von dem Gehäuse 34 abragenden Führungsblatt 49 versehen, welches in eine nicht näher dargestellte Vertikalnut im Innern des Handgerät-Gehäuses 2, den Verschiebeweg bei Hubbetätigung zulassend eingreift.

Die Verrastung zwischen Schrittschaltwerk 11 und Kartusche 3 im Bereich des kartuschenkopfseitigen Kragens 41 ist so gewählt, dass bei einer Entnahme der Kartusche 3 aus dem Gehäuse 2 das Schrittschaltwerk 11 an der Kartusche 3 verbleibend mit ausgezogen wird.

Alternativ besteht auch die Möglichkeit, wie in Fig. 4 schematisch dargestellt, dass das Schrittschaltwerk 11 mit dem Inhaler-Gehäuse 2 verrastet ist. Hierzu überfährt das Schrittschaltwerk 11 im Zuge einer Erstbestückung einen oder mehrere radial nach innen einragende Rastvorsprünge 51 des Gehäuses 2, welche Rastvorsprünge 51 hiernach von radial von dem Schaltwerkgehäuse 34 abragenden Kragenabschnitten 52 unterfangen sind.

Die Rastvorsprünge 51 sind in ihrer Vertikalausrichtung so positioniert, dass die Vertikalverlagerbarkeit des Schrittschaltwerkes 11 bei Hubbetätigung der Kartusche 3 gewährleistet ist.

Durch die gewählte Anordnung formen die Rastvorsprünge 51 Niederhalter aus, welche das Schrittschaltwerk 11 bei einem Abziehen der Kartusche 3 in dem Handgerät-Gehäuse 2 zurückhalten.

Die Darstellung in Fig. 4a zeigt eine weitere Ausführungsform, bauend auf der in Fig. 4 gezeigten Ausgestaltung. So ist zur weiteren Festlegung der Kartusche 3 - neben einer üblichen Klemmhalterung des Ventilrohrs 5 in dem handgerätgehäuseseitigen Stützabschnitt 9 - eine Blockierung der Kartusche 3 im Bereich des oberen Ringteil-Gehäuses 6 vorgesehen. So ragen mantelinnenseitig dieses oberen Gehäuseringteiles 6 schräg abwärts gerichtete Rückhaltefinger 55 ab, welche materialeinheitlich, einstückig mit dem oberen Gehäuseteil 6 gebildet sind. Diese Rückhaltefinger 55 sind so positioniert, dass deren freien Randkanten in der Zuordnungsstellung zur Kartusche 3 in den hinter dem Kartuschenkopf 4 ausgeformten Taillenbereich der Kartusche 3 sperrend eintreten, um so die Kartusche 3 zu blockieren.

Weiter sind die Rückhaltefinger 55 derart ausgeformt, dass diese zumindest bei einer Erstbestückung des Gehäuses 2 mit der Kartusche 3 durch den Kartuschenkopf 4 überlaufen werden können.

Die Kartusche 3 ist somit an dem Handgerätgehäuse 2, insbesondere an dem oberen Gehäuseteil 6 gefesselt.

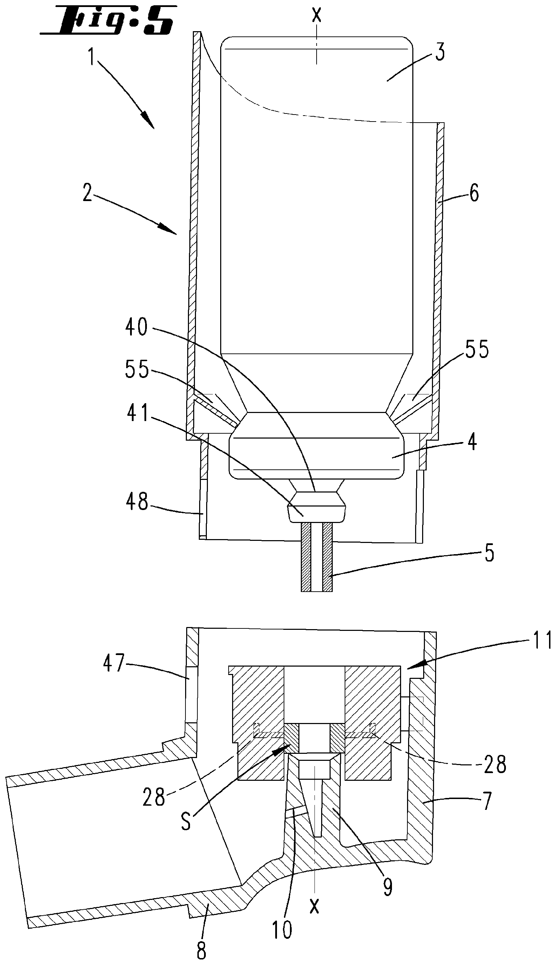

Die Darstellung in Fig. 5 zeigt eine weitere Ausführungsform. In dieser ist auch die Kartusche 3 mittels Rückhaltefingern 55 an dem oberen Gehäuseteil 6 gehaltert. Dieses obere Gehäuseteil 6 ist von dem unteren Gehäuseteil 7, welches das Mundstück 8 ausformt, lösbar, wobei die Trennung der beiden Gehäuseteile 6 und 7 etwa im Bereich der Position des tellerförmigen Schrittschaltwerkgehäuses 34 vorgenommen wird.

In der zusammengesetzten Stellung der beiden Gehäuseteile 6 und 7 sind diese bevorzugt verrastet, wozu ein Gehäuseteil eine Rastnase und das andere Gehäuseteil eine entsprechend platzierte Rastaufnahme aufweist.

Zufolge dieser ermöglichten Trennung ist eine verbesserte Reinigung des Mundstückes 8, insbesondere des den Stufenabschnitt 9 aufweisenden Knickabschnittes erreicht. Dies ist weiter noch dadurch erleichtert, dass das gesamte Schrittschaltwerk 11, welches als kompakte Baugruppe geformt ist, in einfachster Weise aus dem unteren Gehäuseteil 7 entnehmbar ist und so einer gesonderten Reinigung zugeführt werden kann. In dem dargestellten Ausführungsbeispiel sind keine Festlegungsmittel - wie bspw. Rastvorsprünge 51, welche mit Kragenabschnitten 52 zusammenwirken, vorgesehen. Vielmehr ist eine Festlegung des gesamten Schrittschaltwerks 11 in dem unteren Gehäuseteil 7 in Zusammenwirkung mit der Kartusche 3 in der Gebrauchsstellung erreicht, dies unter Ausrichtung des Schrittschaltwerks 11 zwischen dem Stützabschnitt 9 und der zugewandten Stirnfläche des Kartuschenkopfes 4 (wie auch anhand der in Fig. 4a gezeigten Ausführungsform dargestellt).

Auch in dieser Ausführungsform ist das Schrittschaltwerk 11 gegen Drehverlagerung um die Achse x - x durch einen Formschluss zwischen Schrittschaltwerk 11 und unterem Handgerät-Gehäuseteil 7 gesichert.

## Patentansprüche

1. Handgerät (1) zur portionierten Ausgabe sprühfähiger Substanzen, insbesondere Inhaliermedikamenten, mit einer durch Drücken in einem Gehäuse (2) in die Ausgabe-Öffnungs-Stellung verschiebbaren Kartusche (3) und einem beim Öffnungshub der Kartusche (3) von dieser mitbewegten Schaltwerk (11) zum Registrieren und Anzeigen durchgeführter Ausgabebetätigungen, welches Schaltwerk (11) in einem Gehäuse (34) zentral unterhalb einer öffnungsseitigen Stirnwand der Kartusche (3) überlappend zu einem Kartuschen-Ventilrohr (5) angeordnet ist, **gekennzeichnet durch** ein in einem tellerförmigen Gehäuse (34) angeordnetes Schrittschaltwerk (11) mit Schaltgliedern (S, 28, 29, 16, 17, 20, 15, 21, 22, 13, 26), die um in Längsrichtung der Kartusche (3) liegende Achsen umlaufen, wobei eine Skala des Schrittschaltwerkes (11) als konzentrisch zu dem Ventilrohr (5) umlaufender Skalenring (26) gestaltet ist, der über ein Planetenrad-Getriebe (12) von einem ebenfalls konzentrisch zu dem Ventilrohr (5) umlaufenden, von einem Öffnungshub der Kartusche (3) angetriebenen Zahnkranz (16) schrittweise gedreht wird, wobei ein Schrittschaltfinger-Stern (S) vorgesehen ist, eine Nabe (29) des Schrittschaltfinger-Sterns (S) auf einer Stirnfläche eines handgerätgehäuseseitigen Stützabschnitts (9) für das Ventilrohr (5) sitzt, und das Schrittschaltwerkgehäuse (34) bei Durchführung eines Betätigungshubs der Kartusche (3) und damit einhergehender Vertikalverlagerung der Kartusche (3) in Richtung auf den Stützabschnitt (9) über einen Kartuschenkopf (4) mitgeschleppt wird, dies unter Relativverlagerung des Schrittschaltwerkgehäuses (34), des Planetenrad-Getriebes (12) und des Skalenringes (26) zu dem Schrittschaltfinger-Stern (S), welcher eine Abstützung auf dem Stützabschnitt (9) erfährt.

2. Handgerät nach Anspruch 1, **dadurch gekennzeichnet, dass** ein Planetenrad (13) in einer Bohrung (24) des Skalenringes (26) lagert und ein zugehöriges Sonnenrad (14) auf einer unterseitig gezahnten Scheibe (15) sitzt.

3. Handgerät nach einem der vorhergehenden Ansprüche, **dadurch gekennzeichnet, dass** das Planetenrad-Getriebe (12) einen Drehwinkel untersetzt an den Skalenring (26) weitergibt.

4. Handgerät nach einem der vorhergehenden Ansprüche, **dadurch gekennzeichnet, dass** eine auf einer äußeren Mantelfläche des Skalenringes (26) angeordnete und vor einem Sichtfenster (47,48) des Gehäuses (2) laufende Skaleneinteilung (27) jeweils mehreren Einzel-Drehschritten des Planetenrades (13) entspricht.

5. Handgerät nach einem der vorhergehenden Ansprüche, **dadurch gekennzeichnet, dass** ein Zahnkranz (16) mindestens einen von der unteren Randkante schräg aufwärts gerichtet ausgehenden Schlitz (19) zum Eintritt des Schrittschaltfingers (28) aufweist.

6. Handgerät nach einem der vorhergehenden Ansprüche, **dadurch gekennzeichnet, dass** der oder die Schnittschaltfinger (28) schräg aufwärts gerichtet von einer Nabe (29) ausgehen, welche Schrittschaltfinger (28) sich beim Ausgabehub in Richtung einer Ebene senkrecht zur Längsachse bewegen.

7. Handgerät nach einem der vorhergehenden Ansprüche, **dadurch gekennzeichnet, dass** die Nabe (29) vom Ventilrohr (5) durchsetzt ist.

8. Handgerät nach einem der vorhergehenden Ansprüche, **gekennzeichnet durch** eine doppelte Verrastung des als Flachteller gestalteten Schrittschaltwerkgehäuses (34) derart, dass neben der Verrastung an einem Kragen (41) der Kartusche (3) eine zweite stärkere Verrastung im Handgerätgehäuse (2) erfolgt.

9. Handgerät nach Anspruch 8, **dadurch gekennzeichnet, dass** die stärkere Verrastung im Handgerätgehäuse (2) nach Entfernung der Kartusche (3) aus dem Handgerätgehäuse (2) für sich auflösbar ist.

10. Handgerät nach einem der vorhergehenden Ansprüche, **dadurch gekennzeichnet, dass** die Kartusche (3) durch Rückhaltefinger (55), die schräg abwärts weisend in dem Handgerätgehäuse (2) ausgebildet sind, gegen Abziehen blockiert ist.

11. Handgerät nach einem der vorhergehenden Ansprüche, **dadurch gekennzeichnet, dass** ein oberes Handgerätgehäuseteil (6) vom Mundstück (8) etwa im Bereich der Position des tellerförmigen Schrittschaltwerkgehäuses (34) trennbar ist.

## Claims

1. Hand-held device (1) for delivering portions of sprayable substances, in particular inhaler medicaments, having a cartridge (3) which is displaceable in a housing (2) into the open position for delivery by pressing, and an indexing mechanism (11) which is moved along with and by the cartridge (3) during the opening stroke thereof, for registering and displaying delivery actuations which have been carried out, which indexing mechanism (11) is disposed in a housing (34) centrally below the opening-side end wall of the cartridge (3), overlappingly with respect to the valve tube (5) of the cartridge, **characterized by** a step-by-step indexing mechanism (11) which is disposed in a plate-shaped housing (34) and has indexing members (S, 28, 29, 16, 17, 20, 15, 21, 22, 13, 26) which revolve about axes lying in the longitudinal direction of the cartridge (3), wherein, a scale of the step-by-step indexing mechanism (11) is configured as a graduated ring (26) which revolves concentrically with the valve tube (5) and is rotated step-by-step by a toothed ring (16), which likewise revolves concentrically with the valve tube (5) and is driven by the opening stroke of the cartridge (3), via a planet-wheel gear mechanism (12), wherein, a step-by-step indexing-finger star (S) is provided, a hub (29) of the step-by-step indexing-finger star (S) is seated on an end surface of a supporting portion (9) for the valve tube (5), which supporting portion is on the hand-held device-housing side and the step-by-step indexing-mechanism housing (34) when an actuating stroke of the cartridge (3) is carried out together with an associated vertical displacement of the cartridge (3) in the direction of the supporting portion (9), the step-by-step indexing-mechanism housing (34) is carried along via a cartridge head (4), with displacement of the housing (34), the planet-wheel gear mechanism (12) and the graduated ring (26) relative to the step-by-step indexing-finger star (S), which receives support on the supporting portion (9).

2. Hand-held device according to claim 1, **characterized in that** a planet wheel (13) is mounted in a hole (24) in the graduated ring (26) and the associated sun wheel (14) is seated on a disk (15) having teeth on the lower side.

3. Hand-held device according to one of the preceding claims, **characterized in that** the planet-wheel gear mechanism (12) passes on the angle of rotation to the graduated ring (26) with a step-down ratio.

4. Hand-held device according to one of the preceding claims, **characterized in that** a graduation (27), which is disposed on an outer circumferential surface of the graduated ring (26) and runs in front of a viewing window (47, 48) of the housing (2), corresponds in each case to a plurality of individual rotary steps of the planet wheel (13).

5. Hand-held device according to one of the preceding claims, **characterized in that** a toothed ring (16) has at least one slot (19), which extends from the lower peripheral edge in a direction obliquely upwards, for entry of the step-by-step indexing finger (28).

6. Hand-held device according to one of the preceding claims, **characterized in that** the step-by-step indexing finger or fingers (28) extend from a hub (29) in a direction obliquely upwards, which step-by-step indexing fingers (28) move in the direction of a plane perpendicular to the longitudinal axis during the delivery stroke.

7. Hand-held device according to one of the preceding claims, **characterized in that** the valve tube (5) passes through the hub (29).

8. Hand-held device according to one of the preceding claims, **characterized by** a double latching of the step-by-step indexing-mechanism housing (34), which is configured as a flat plate, in such a manner that, in addition to a latching to the collar (41) of the cartridge (3), a second stronger latching takes place in the hand-held device housing (2).

9. Hand-held device according to one of the preceding claims, **characterized in that** the stronger latching in the hand-held device housing (2) can itself be released after removal of the cartridge (3) from the hand-held device housing (2).

10. Hand-held device according to one of the preceding claims, **characterized in that** the cartridge (3) is blocked against being pulled off by means of retaining fingers (55) which are formed pointing obliquely downwards in the hand-held device housing (2).

11. Hand-held device according to one of the preceding claims, **characterized in that** an upper hand-held device housing part (6) can be separated from the mouthpiece (8) approximately in the region of the position of the plate-shaped step-by-step indexing-mechanism housing (34).

## Revendications

1. Dispositif manuel (1) de distribution fractionnée de substances pulvérisables, en particulier des médicaments à inhaler, comprenant une cartouche (3) qui, par appui, est déplaçable dans un boîtier (2) dans la position d'ouverture-distribution et un mécanisme d'indexage (11) qui lors de la course d'ouverture de la cartouche est déplacé par celle-ci pour indexer et indiquer les actionnements de distribution réalisés, lequel mécanisme d'indexation (11) est agencé dans un boîtier (34) centralement sous une paroi frontale de la cartouche (3) du côté de son ouverture, en chevauchement à un tube de valve (5) de la cartouche, **caractérisé par** un mécanisme pas-à-pas (11) agencé dans un boîtier en forme d'assiette (34) et ayant des organes d'indexation (S, 28, 29, 16, 17, 20, 15, 21, 22, 13, 26) qui tournent autour d'axes s'étendant dans la direction longitudinale de la cartouche (3), dans lequel une échelle graduée du mécanisme pas à pas (11) est réalisée sous la forme d'une bague graduée (26) qui tourne concentriquement par rapport au tube de valve (5) et qui est entraîné pas-à-pas par un engrenage planétaire (12) par le biais d'une couronne dentée (16) qui tourne également de manière concentrique par rapport au tube de valve (5) et qui est entraînée par une course d'ouverture de la cartouche (3), dans lequel une étoile à doigts d'indexation pas-à-pas (S) est prévue, un moyeu (29) de l'étoile à doigts d'indexation pas-à-pas (S) est placé sur une face frontale d'une partie de support (9) du tube de valve (5) située côté boîtier du dispositif manuel, et le boîtier (34) du mécanisme pas à pas est entraîné par une tête (4) de la cartouche lorsque la cartouche (3) effectue une course d'actionnement et que la cartouche (3) se déplace ainsi verticalement dans la direction de la partie de support (9), ceci avec un déplacement relatif du boîtier (34) du mécanisme pas à pas, de l'engrenage planétaire (12) et de la bague graduée (26) vers l'étoile à doigts d'indexation pas-à-pas (S) qui est en appui sur la partie de support (9) .

2. Dispositif manuel selon la revendication 1, **caractérisé en ce qu'**une roue satellite (13) est montée dans un alésage (24) de la bague d'indexation (26) et une roue planétaire associée (14) est disposée sur un disque (15) denté sur le dessous.

3. Dispositif manuel selon l'une des revendications précédentes, dans lequel l'engrenage planétaire (12) transmet de manière réduite un angle de rotation à la bague graduée (26).

4. Dispositif manuel selon l'une des revendications précédentes, **caractérisé en ce qu'**une graduation (27) agencée sur une surface périphérique extérieure de la bague graduée (26) et se déplaçant devant une fenêtre de visualisation (47, 48) du boîtier (2) correspond à chaque fois à plusieurs pas de rotation individuels de la roue satellite (13).

5. Dispositif manuel selon l'une des revendications précédentes, **caractérisé en ce qu'**une couronne dentée (16) présente au moins une fente (19) qui s'étend obliquement vers le haut à partir du bord inférieur pour la pénétration du doigt d'indexation (28).

6. Dispositif manuel selon l'une des revendications précédentes, **caractérisé en ce que** le ou les doigts d'indexation (28) s'étendent obliquement vers le haut à partir d'un moyeu (29), lesquels doigts d'indexation (28) se déplacent dans la direction d'un plan perpendiculaire à l'axe longitudinal pendant la course de distribution.

7. Dispositif manuel selon l'une des revendications précédentes, **caractérisé en ce que** le moyeu (29) est traversé par le tube de valve (5).

8. Dispositif manuel selon l'une des revendications précédentes, **caractérisé par** un double encliquetage du boîtier (34) du mécanisme pas à pas qui est réalisé sous la forme d'une assiette plate, de manière à procurer, outre l'encliquetage sur un col (41) de la cartouche (3), un deuxième encliquetage plus fort dans le boîtier (2) du dispositif manuel.

9. Dispositif manuel selon la revendication 8, **caractérisé en ce que** l'encliquetage plus fort dans le boîtier (2) du dispositif manuel peut être libéré de lui-même après retrait de la cartouche (3) hors du boîtier (2) du dispositif manuel.

10. Dispositif manuel selon l'une des revendications précédentes, **caractérisé en ce que** la cartouche (3) est bloquée contre le retrait par des doigts de retenue (55) formés dans le boîtier (2) du dispositif manuel et qui s'étendent obliquement vers le bas.

11. Dispositif manuel selon l'une des revendications précédentes, **caractérisé en ce qu'**une partie supérieure (6) du boîtier du dispositif manuel est séparable de l'embout (8) approximativement dans la région de la position du boîtier (34) du mécanisme pas-à-pas en forme d'assiette.
